# EUROPÄISCHE PATENTSCHRIFT

(11) **EP 1 237 525 B1**
(45) Veröffentlichungstag und Bekanntmachung des Hinweises auf die Patenterteilung: **03.09.2008**
(21) Anmeldenummer: 00991789.9
(22) Anmeldetag: 15.12.2000
(51) Int. Cl.: A61K 6/09

(54) **VERBESSERTE DENTALMATERIALIEN**
IMPROVED DENTAL MATERIALS
MATERIAUX DENTAIRES AMELIORES

(30) Priorität: 17.12.1999 DE 19961341
(43) Veröffentlichungstag der Anmeldung: 11.09.2002
(73) Patentinhaber: 3M ESPE AG, 82229 Seefeld (DE)
(72) Erfinder: LEHMANN, Thomas, 84489 Burghausen (DE); SOGLOWEK, Wolfgang, 86911 Diessen-Obermühlhausen (DE); HECHT, Reinhold, 86916 Kaufering (DE)
(74) Vertreter: Brem, Roland
(86) Internationale Anmeldenummer: PCT/EP2000/012774
(87) Internationale Veröffentlichungsnummer: WO 2001/043700

(56) Entgegenhaltungen:
- EP-A- 0 231 805
- EP-A- 0 630 640
- WO-A-96/15179
- WO-A-99/40884
- DE-A- 19 841 205

## Beschreibung

Die vorliegende Erfindung betrifft Zusammensetzungen zur Herstellung von Dentalmaterialien mit verbesserten mechanischen Eigenschaften und die Verwendung solcher Dentalmassen, insbesondere als provisorische bzw. temporäre Kronen, Brücken, Füllungen, Inlays und Onlays.

Bei der Herstellung von provisorischen bzw. temporären Kronen, Brücken, Inlays und Onlays hat man es je nach Anwendungszweck mit dünnfließenden bis zähplastischen Massen auf Basis ethylenisch ungesättigter Verbindungen zu tun, die mit organischen und/oder anorganischen Füllstoffen versehen sein können und durch Polymerisation aushärten (im weiteren "provisorische K+B-Materialien" genannt).

Zwei verschiedene Klassen an provisorischen K+B Materialien sind gängig: Zum einen sogenannte Polymethylmethacrylat-Materialien (PMMA-Materialien) und zum anderen sogenannte Composite-Materialien.

Die PMMA-Materialien bestehen aus einem Pulver/Flüssigkeitssystem, wobei es sich bei dem Pulver im wesentlichen um ein PMMA-Pulver mit Farbstoffen und Initiatorbestandteilen handelt und die Flüssigkeit hauptsächlich aus Methylmethacrylat und/oder iso-Butylmethacrylat und Initiatoren sowie Stabilisatoren besteht.

Aus PMMA-Material hergestellte Kronen und Brücken zeichnen sich durch eine geringe Bruchanfälligkeit und gute elastische Eigenschaften aus. Ein entscheidender Nachteil dieser Materialien ist aber, dass sie sich bei Belastung irreversibel verformen.

So steht mit den PMMA-Materialien ein wenig bruchanfälliges, aber dafür leicht permanent verformbares Material, was bei zu hoher Belastung für den Patienten unbrauchbar wird, zur Verfügung. Zusätzlich sind Materialien auf PMMA-Basis physiologisch bedenklich, da sie toxische Acrylat-Systeme enthalten.

Die Composite-Materialien bestehen üblicherweise aus Paste/Paste Systemen, die entweder per Hand angemischt werden oder in einer Doppelkammerkartusche vorliegen und mittels eines Dispensers und einer statischen Mischvorrichtung, wie in der EP-A-0 232 733 und der EP-A-0 261 466 beschrieben, angemischt werden. Bei den in dieser Materialklasse eingesetzten Monomeren handelt es sich hauptsächlich um difunktionelle ethylenisch ungesättigte Verbindungen, die beim Aushärten einen hohen Vernetzungsgrad der entstehen Polymermatrix bewirken. Als Füllstoffe werden im wesentlichen Glaspulver, Kieselgele und Quarze eingesetzt.

Durch den hohen Vernetzungsgrad und die harten Füllstoffe werden harte und spröde Materialien erhalten, die bei hoher Belastung nicht fließen, sondern brechen.

Mit den Composite-Materialien steht daher ein sehr formstabiles, dafür aber bruchanfälliges Material, was ebenfalls bei zu hoher Belastung für den Patienten unbrauchbar wird, zur Verfügung.

Aus dem Stand der Technik sind als Monomere für Composite-Materialien auch Urethan(meth)acrylate, im wesentlichen 7,7,9-Trimethyl-4,13-dioxo-3,14,-dioxa-5,12-diazahexadecan-1,16-dioxy-dimethacrylat (UDMA, beispielsweise Plex 666-1, Fa. Röhm, Darmstadt), beschrieben worden.

Unter Acrylaten bzw. (Meth)acrylaten werden im Rahmen dieser Schrift generell Acrylate und/oder Methacrylate verstanden.

UDMA verbessert geringfügig die Bruchanfälligkeit, jedoch handelt es sich bei den mit UDMA hergestellten Compositen immer noch um spröde Materialien, die speziell in dünnen Bereichen oder bei der Anwendung in Brücken generell bruchanfällig sind.

Um die Bruchanfälligkeit weiter zu verringern hat es verschiedene erfolglose. Versuche gegeben:
- Das Einarbeiten von Polymeren, weiches aufgrund des starken Quellverhaltens in der Monomermatrix zu einem starken Viskositätsanstieg führt.
- Der Einsatz von Weichmachern, weicher mit einem starken Abfall der mechanischen Eigenschaften, wie der Druck- und Biegefestigkeit, sowie der Auslösung schädlicher Substanzen in Körpefflüssigkeiten einhergeht.
- Der Einsatz von hochmolekularen, niedrigfunktionellen Monomeren, wie Polyethylenglycoldiacrylat, welcher zwar die Bruchanfälligkeit verbessert, aber die mechanischen Eigenschaften stark verringert sowie zu einem starken Temperaturanstieg bei der Aushärtung führt und daher bei der Applikation im Mund nicht brauchbar ist.

Zusammengefasst führt also keine der bisher bekannten Methoden zu einer brauchbaren Verbesserung der Bruchanfälligkeit und Formstabilität von provisorischen K+B-Materialien auf Composite-Basis.

Die DE 198 41 205 A beschreibt lichthärtende Harzmassen zur Ortodontie, welche neben Füllstoffen, Initiatorsystemen und Weichmachern Urethanbindungen enthaltende (Meth)acrylate mit einem Molekulargewicht von 300 bis 5000 g/mol und mindestens einer ungesättigten Doppelbindung enthalten, wobei Mischungen solcher Verbindungen unterschiedlichen Molekulargewichts nicht ausgeschlossen sind, sowie zusätzlich Urethanbingungsfreie (Meth)acrylate mit einem mittleren Molekulargewicht von 100 bis 300 g/mol und mindestens einer ungesättigten Doppelbindung. Bezüglich der zuletzt genannten Verbindungen wird in der Beschreibung in den Zeilen 12 bis 14 von Seite 3 gesagt, daß das Molekulargewicht 300 nicht übersteigen darf, da sonst das erhaltene gehärtete Material spröde und in ungeeigneter Weise in der Haltbarkeit mangelhaft ist.

Die WO 99/40884 beschreibt Dentalmaterialien auf der Basis von polymerisierbaren Acrylmonomeren, Polymerisationsinitiatoren und inhibitoren sowie Füllstoffen. Zusammensetzungen, die Urethan(meth)acrylate mit unterschiedlichem Molekulargewichten sowie ungesättigte Verbindungen, die kein Urethan(meth)acrylat darstellen enthalten, sind dort nicht offenbart Die in den Beispielen beschriebenen Zusammensetzungen enthalten ein einziges Urethanmethacrylat.

Aufgabe der Erfindung ist es, ein Dentalmaterial auf Composite-Basis zur Verfügung zu stellen, das eine verringerte Bruchanfälligkeit bei gleichzeitig hoher Formstabilität aufweist.

Gelöst wurde diese Aufgabe durch Zusammensetzungen, enthaltend:
(a) 14 bis 60 Gew.-%, bevorzugt 20 bis 50 Gew.-% Füllstoffe,
(b) 3 bis 35 Gew.-%, bevorzugt 5 bis 25 Gew.-% mindestens einer einfach oder mehrfach ethylenisch ungesättigten Verbindung, die kein Urethan(meth)-acrylat ist und ein Molekulargewicht zwischen 300 und 5000 g/mol aufweist,
(c) 0,1 bis 5 Gew.-%, bevorzugt 0,1 bis 4 Gew.% mindestens eines Initiatorsystems, das befähigt ist eine radikalische Reaktion zu starten,
(d) 1 bis 40 Gew.-%, bevorzugt 2 bis 30 Gew.-% Weichmacher,
(e) 0 bis 10 Gew.-%, bevorzugt 0,000001 bis 8 Gew.-% Hilfsstoffe wie Farbstoffe, Pigmente, Stabilisatoren, Lösungsmittel, rheologische Additive, wie Fließverbesserer und/oder Verzögerer, und
(f) 30 bis 81,9 Gew.-%, bevorzugt 35 bis 73 Gew.-% mindestens zwei im Molekulargewicht unterschiedlichen Urethan(meth)acrylate, wobei diese in einem Molekulargewichtsverhältnis von 1,5 ; 1 bis 50 : 1 vorliegen und das höhermolekulare Urethan(meth)acrylat ein Molekulargewicht zwischen 1.000 und 20.000 g/mol und das niedrigermolekulare Urethan(meth)acrylat ein Molekulargewicht zwischen 300 und 1.000 g/mol aufweisen.

Die erfindungsgemäßen Massen eignen sich besonders für den Einsatz bei der Herstellung von provisorischen bzw. temporären Kronen, Brücken, Füllugen, Inlays und Onlays.

Überraschenderweise wurde gefunden, dass Materialien, hergestellt aus den erfindungsgemäßen Zusammensetzungen, eine in hohem Masse verbesserte Bruchanfälligkeit bei gleichzeitig hoher Formstabilität zeigen. Die erfindungsgemäßen Materialien haben verbesserte elastische Eigenschaften bei weitgehendem Erhalt der mechanischen Stabilität.

Die Begriffe "umfassend" oder "enthaltend" sollen im Rahmen dieser Schrift stets eine nicht-abgeschlossene Aufzählung einleiten.

Der Umstand, dass das Wort "ein" vor Nennung eines Merkmals verwendet wird, schließt nicht aus, dass die genannten Merkmale mehrmals vorhanden sein können, im Sinne von "mindestens ein".

Komponente (a) umfasst übliche Füllstoffe für Dentalwerkstoffe, beispielsweise Glas- und Quarzpulver, Kieselgele, pyrogene hochdisperse Kieselsäuren, unlösliche Kunststoffe, schwer lösliche Fluoride, wie in der US-A-5,824,720 beschrieben, sowie Mischungen dieser Komponenten. Diese Füllstoffe können durch geeignete Zusätze, wie beispielsweise barium- oder strontiumhaltige Gläser, oder auch durch Verbindungen, wie YF₃, röntgenopak sein. Als die Thixotropie beeinflussende Füllstoffe sind beispielsweise pyrogene hochdisperse Kieselsäuren geeignet. Weiterhin können auch lösliche organische Polymerisate, wie Polyvinylacetat sowie dessen Copolymere in dem Maße zugesetzt werden, dass die Viskosität der Pasten einen Einsatz in handelsüblichen Kartuschensystemen, wie sie in der EP-A-0 232 733 und EP-A-0 261 466 beschrieben sind, erlaubt.

Auch Christobalit, Calciumsilikat, Zirkoniumsilikat, Molekularsiebe, wie Natriumaluminiumsilikat, Metalloxidpulver, wie Aluminium- oder Zinkoxide bzw. deren Mischoxide, Bariumsulfat, Yttriumfluorid, Calciumcarbonat, Gips und Kunststoffpulver sind beispielsweise als Füllstoffe geeignet.

Die genannten Füllstoffe können beispielsweise durch eine Behandlung mit Organosilanen bzw. -siloxanen oder durch die Veretherung von Hydroxylgruppen zu Alkoxygruppen hydrophobiert sein.

Es hat sich gezeigt, dass die Verwendung von rein organischen, unlöslichen Füllstoffen sich nicht zur Lösung der vorliegenden Aufgabe eignet, da ästhetisch minderwertige Dentalmaterialien mit ungenügenden physikalischen Werten erhalten werden.

Bei Komponente (b) handelt es sich um mindestens eine ein- oder mehrfach ethylenisch ungesättigte Verbindung, die kein Urethan(meth)acrylat ist und bevorzugt eine Viskosität kleiner 5 Pa.s bei 23°C (Kegel-Platte-Viskosimeter). Geeignet sind aber auch Verbindungen mit einer anderen Viskosität. Die Verbindungen weisen ein Molekulargewicht zwischen 300g/mol und 5000g/mol, bevorzugt zwischen 300 g/mol und 1000 g/mol auf.

Besonders geeignet als Komponente (b) sind Acrylsäure- und/oder Methacrylsäureester einer ethoxylierten und/oder propoxylierten Verbindung, eines Polyethers oder eines Alkylpolyols.

Hierbei kann es sich beispielsweise um Di(meth)acrylate von Alkylverbindungen, bevorzugt mit 2 bis 20 C-Atomen, handeln. Bevorzugt sind Di(meth)acrylate des Hexandiols (6 C-Atomen), Octandiols (8 C-Atomen). Nonandiols (9 C-Atomen), Decandiols (10 C-Atomen) und Eicosandiols (20 C-Atomen).

Geeignet sind auch Di(meth)acrylate von ethoxylierten und/oder propoxylierten Verbindungen, beispielsweise des Ethylenglycols, der Polyethylenglycole, der Polypropylenglycole, Polyethylen-co-propylenglycole.
Geeignet sind ferner Di(meth)acrylate des ethoxylierten Bisphenol A, beispielsweise 2,2'-Bis(4-(meth)acryloxy-tetraethoxyphenyl)propane.

Als Komponente (c) enthalten die erfindungsgemäßen Zusammensetzungen mindestens ein Initiatorsystem, das geeignet ist, Radikale zu erzeugen. Geeignet sind prinzipiell Redoxsysteme oder strahlenhärtende Systeme oder auch Mischungen verschiedener Katalysatorsysteme.

Als Redoxsystem kann beispielsweise ein Initiatorsystem bestehend aus einer Amin- und einer Peroxid-Komponente, wie in der DT-PS-97 50 72 beschrieben, sein. Die Polymerisation wird hier durch die Peroxidverbindung gestartet. Als Beschleuniger der Polymerisation wird beispielsweise ein tertiäres Amin eingesetzt. Ein weiteres geeignetes System wird auch von Albert Groß in "Quintessenz der Zahntechnik", 1977, 7, Referat Nr. 293, beschrieben. Üblicherweise wird die Amin-Komponente dabei in eine Paste, die sogenannte Basispaste, eingearbeitet. Diese Basispaste enthält meist auch die zur Polymerisation vorgesehenen Monomere. Die Peroxid-Komponente wird in eine weitere Paste, die sogenannte Katalysatorpaste, eingearbeitet. Die räumliche Trennung der beiden Initiatorkomponenten ist erforderlich, um eine vorzeitige Aushärtung der Monomeranteile zu vermeiden.

Auch in der DE-A-95 56 33 wird ein ähnliches Initiatorsystem für die Polymerisation von ungesättigten Kohlenwasserstoffen beschrieben, das Schwermetalle sowie eine Amin- und eine Sulfon-Komponente enthält. Auch in der EP-A-0 374 824 wird ein Initiatorsystem mit einer organischen Peroxidverbindung und einem tertiären aromatischen Amin als Aktivator (Beschleuniger) genannt. Beispielhaft können all diese Systeme alleine oder in Kombination in den erfindungsgemäßen Zusammensetzungen eingesetzt werden.

Eine günstigere Temperaturentwicklung und auch bessere Farbstabilität weisen die Initiatorsysteme auf, die beispielsweise in der DT-C-14 95 520 beschrieben werden. Die Zusammensetzung aus der DT-C-14 95 520 polymerisiert bei niedriger Temperatur in kurzer Zeit und ohne Anwendung von externer Energie. Die beschriebenen Systeme enthalten Barbitursäurederivate bzw. Malonylsulfamide, ionogen gebundenes Halogen und/oder eine Schwermetallverbindung und/oder organische Peroxide. Auch EP-A-0 374 824 beschreibt ein derartiges Initiatorsystem aus Barbitursäurederivat, Peroxid, Schwermetallverbindung und ionogenem Halogen.

Weiterhin kann die erfindungsgemäße polymerisierbare Zusammensetzung ein Photoinitiatorsystem enthalten. Dieses kann zusätzlich neben mindestens einem der anderen Initiatorsysteme oder als alleiniges Initiatorsystem vorliegen. Geeignete Photoinitiatoren sind zum Beispiel die in der US-A-47 92 632 und US-A-47 37 593 beschriebenen Bisacylphosphinoxide.

Wesentlich für die Erfindung ist es, dass die Initiatorsysteme keine Zuführung von Energie in Form von Wärme benötigen, um Radikale zu erzeugen. Wärmehärtende Systeme sind für den Einsatz als Dentalmaterialien, insbesondere im Mund des Patienten aushärtenden Materialien, unbrauchbar.

Als Komponente (d) können die erfindungsgemäßen polymerisierbaren Zusammensetzungen übliche Weichmacher, bevorzugt mit einer Viskosität kleiner als 10 Pa.s bei 23°C (Kegel-Platte-Viskosimeter), enthalten. Diese sind beispielweise Polyethylenglykolderivate, Polypropylenglycole, niedermolekulare Polyester, Dibutyl-, Dioctyl-, Dinonyl-, Diphenyl-Phthalat, Di(iso-nonyladipat), Tricresylphosphat, Paraffinöle und Siliconöle.

Als Komponente (e) können Hilfsstoffe, wie Farbstoffe, Pigmente, Stabilisatoren, Lösungsmittel und/oder rheologische Additive, wie Fließverbesserer zugesetzt werden. Weiterhin können zur Verlängerung der Abbindezeiten beispielsweise die in der EP-A-0 374 824 als Komponente {d} beschriebenen Vinylverbindungen als Verzögerer eingesetzt werden.

Komponente (f) besteht aus mindestens zwei im Molekulargewicht unterschiedlichen Urethan(meth)acrylaten mit einem Molekulargewichtsverhältnis von 1,5 : 1 bis 50 : 1, bevorzugt 1,5 : 1 bis 20 : 1, besonders bevorzugt 1,5:1 bis 10 : 1 und ganz besonders bevorzugt 1,5 :1 bis 5 : 1. Besonders vorteilhaft ist es, wenn in den erfindungsgemäßen Zusammensetzungen die Acrylate dieser Komponente mindestens 50 Gew.-%, bevorzugt mindestens 60 Gew.-%, besonders bevorzugt mindestens 70 Gew.-% der gesamten ethylenisch ungesättigten Verbindungen [(b) + (f)] ausmachen.

Die höhermolekularen Acrylate dieser Komponente weisen ein Molekulargewicht zwischen 1.000 g/mol und 20.000 g/mol, bevorzugt zwischen 1.000 g/mol und 15.000 g/mol und besonders bevorzugt zwischen 1.000 g/mol und 10.000 g/mol auf.

Die niedermolekularen Acrylate dieser Komponente weisen ein Molekulargewicht zwischen 300 g/mol und 1.000 g/mol auf.

Bei dem höhermolekularen Urethan(meth)acrylat der Komponente (f) kann es sich um ein Polyester-, Polyether-, Polybutadien- und/oder Polycarbonaturethanoligomer(meth)acrylat handeln.

Unter einem Polyether-urethanoligomer(meth)acrylat wird beispielsweise eine Verbindung verstanden, die mindestens Polyether-, Urethan- und (Meth)acrylat-Gruppierungen enthält.

Diese Urethanoligomer(meth)acrylate sind zugänglich, indem ein Polyester-, Polyether-, Polybutadien- und/oder Polycarbonat-Diol (Diolkomponente) mit einem aliphatischen, cycloaliphatischen und/oder aromatischen Diisocyanat, beispielsweise 1,6-Hexamethylendiisocyanat (HDI), 2,4,4-Trimethylhexamethylen-1,6-diisocyanat (TMDI), Tetramethylendiisocyanat, isophorondiisocyanat, 4,4'-Dicyclohexylmethandiisocyanat, 1,4-Phenylendiisocyanat, 2,6- und 2,4-Toluoldiisocyanat, 1,5-Naphthylendiisocyanat, 2,4'- und 4,4'-Diphenylmethandiisocyanat (Diisocyanatkomponente) unter Amin oder Zinnkatalyse umgesetzt wird (C. Hepburn, "Polyurethane Elastomers", 2nd Ed, Elsevier Applied Science, London and New York, 1992). Wird hierbei mit einem molaren Überschuss an Diolkomponente im Vergleich zur Diisocyanatkomponente gearbeitet, bleiben endständige OH-Gruppen übrig, die mit einer ethylenisch ungesättigten Säure, wie Acrylsäure oder Methacrylsäure bzw. einem ihrer Derivate, verestert werden. Wird mit einem molaren Überschuss an Diisocyanatkomponente im Vergleich zur Diolkomponente gearbeitet, bleiben endständige Isocyanatgruppen übrig, die mit einem Hydroxyalkyl- und/oder Hydroxyaryl-(meth)acrylat und/oder -di(meth)acrylat und/oder -tri(meth)acrylat, wie beispielsweise 2-Hydroxyethylacrylat (HEA), 2-Hydroxyethylmethacrylat (HEMA), 3-Hydroxypropylmethacrylat (HPMA), 3-Hydroxypropylacrylat (HPA) Glycerindimethacrylat und/oder Glycerindiacrylat, umgesetzt werden.

Die Herstellung von zuvor genannten Urethan(meth)acrylaten ist beispielsweise aus C. Hepburn, "Polyurethane Elastomers", 2nd Ed, Elsevier Applied Science, London and New York, 1992, entnehmbar.

Einsetzbare Polycarbonatpolyole sind beispielsweise Produkte, die durch Umsetzung von Diolen, wie 1,3-Propandiol, 1,4-Butandiol, 1,6-Hexandiol, Diethylenglykol, Neopentylglycol, Trimethyl-1,6-Hexandiol, 3-Methyl-1,5-pentandiol und/oder Tetraethylenglykol, mit Diarylcarbonaten, wie Diphenylcarbonat, oder mit Phosgen resultieren, wie in der US-A-4,533,729, DE-A-169 40 80, DE-A-271 43 03 oder EP-B-0 343 572 beschrieben.

Einsetzbare Polyetherpolyole umfassen beispielsweise Produkte, die durch Polymerisation eines cyclischen Oxids, beispielsweise Ethylenoxid, Propylenoxid oder Tetrahydrofuran, oder durch Addition eines oder mehrerer dieser Oxide an polyfunktionelle Initiatoren, wie Wasser, Ethylenglykol, Propylenglykol, Diethylenglykol, Cyclohexandimethanol, Glycerin, Trimethylolpropan, Pentaerythrit oder Bisphenol A, zugänglich sind. Besonders geeignete Polyetherpolyole sind Polyoxypropylendiole und triole, Poly(oxyethylen-oxypropylen)diole und -triole, die durch gleichzeitige oder aufeinanderfolgende Addition von Ethylen- und Propylenoxid an geeignete Initiatoren erhalten werden, sowie Polytetramethylenetherglykole, die durch Polymerisation von Tetrahydrofuran entstehen.

Geeignete Polyetherpolyole sind beispielsweise auch unter der Bezeichnung "Desmophen" von der Fa. Bayer, Leverkusen, erhältlich.

Bei den Polyesterpolyolen handelt es sich um Umsetzungsprodukte von niedermolekularen Polyolen mit niedermolekularen Polycarbonsäuren.

Hierfür geeignete niedermolekulare Polyole bzw. Polyolgemische sind beispielsweise Ethylenglykol, Propylenglykol, 1,4-Butandiol, 1,6-Hexandiol, Diethylenglykol, Triethylenglykol, Neopentylglykol, 1,4-Bis(hydroxymethyl)-cyclohexan, Dipropylenglykol. Als höherfunktionelle Polyole, die anteilig, beispielsweise von 0 bis 20 Gew.-%, mitverwendet werden können, um Verzweigungen in das Polyestermolekül einzubringen, sind beispielsweise Glycerin, Trimethylolpropan oder Pentaerythrit geeignet. Besonders bevorzugt sind 1,6-Hexandiol und Neopentylglykol.

Die niedermolekularen Polycarbonsäuren können beispielsweise aliphatischer, cycloaliphatischer, aromatischer und/oder heterocyclischer Natur sein. Anstelle der freien Polycarbonsäuren können auch entsprechende Polycarbonsäureanhydride oder Polycarbonsäureester mit niederen Alkoholen eingesetzt werden. Beispielhaft seien genannt: Bernsteinsäure, Adipinsäure, Sebacinsäure, Azelainsäure, Phthalsäure, Isophthalsäure, Phthalsäureanhydrid, Tetrahydrophthalsäureanhydrid, Glutarsäureanhydrid, Maleinsäure, Maleinsäureanhydrid, Fumarsäure, Terephthalsäuredimethylester. Besonders bevorzugt ist Adipinsäure.

Geeignete Polyesterpolyole sind beispielsweise auch unter der Bezeichnung "Desmophen" (Fa. Bayer, Leverkusen) erhältlich.

Polyester, die beispielsweise durch Polymerisation von Lactonen, wie Caprolacton, in Verbindung mit einem Polyol zugänglich sind, können ebenfalls eingesetzt werden. Polyesteramidpolyole können durch anteilige Verwendung von Aminoalkoholen, wie Ethanolamin, im Polyesterbildungsgemisch erhalten werden.

Geeignete Polyolefinpolyole sind beispielsweise Butadienhomo- und - copolymere mit endständigen Hydroxylgruppen, die beispielsweise bei der Firma BFGoodrich Speciality Chemicals, Cleveland, Ohio erhältlich sind.

Käufliche Urethan(meth)acrylate mit einem Molekulargewicht größer als 1.200 g/mol sind beispielsweise folgende, wobei die Liste keinen Anspruch auf Vollständigkeit erhebt und die Erfindung in keinster Weise limitierend zu verstehen ist:
Urethan-MA 92-456, Urethan-A 98-446, Genomer 4269, Genomer 4215, Genomer 4246 (Fa. Rahn AG, Zürich), Ebecryl 230, Ebecryl 270, Ebecryl 930, (UCB Chemicals, Kerpen), BR-304, BR-374, BR-3731, BR-582E, BR-7432, BR-204 (Bomar Specialities Co., Winsted).

Das erfindungsgemäß verwendete Urethan(meth)acrylat mit dem kleineren Molekulargewicht der Komponente (f) kann beispielsweise ein Umsetzungsprodukt sein aus einem difunktionellen, beispielsweise aliphatischen, cycloaliphatischen und/oder aromatischen, Isocyanat, wie 1,6-Hexamethylendiisocyanat (HDI), 2,4,4-Trimethyl-hexamethylen-1,6-diisocyanat (TMDI), Butylisocyanat, Tetramethylendiisocyanat, Isophorondiisocyanat, 4,4'-Dicyclohexylmethandiisocyanat, 1,4-Phenylendiisocyanat, 2,6- und 2,4-Toluoldiisocyanat, 1,5-Naphthylendiisocyanat, 2,4'- und 4,4'-Diphenylmethandiisocyanat, unter Amin- oder Zinnkatalyse mit einem Hydroxyalkyl- und/oder Hydroxyaryl-(meth)acrylat und/oder -di(meth)acrylat und/oder -tri(meth)acrylat, wie 2-Hydroxyethylacrylat (HEA), 2-Hydroxyethylmethacrylat (HEMA), 3-Hydroxypropylmethacrylat (HPMA); 3-Hydroxypropylacrylat (HPA) Glycerindimethacrylat und/oder Glycerindiacrylat.

Üblicherweise werden Dentalmaterialien aus den erfindungsgemäßen Zusammensetzungen als zweikomponentige Paste-Paste-Systeme in Applikationssystemen bzw. -geräten, wie Kartuschen der Firma Mixpack, Rotkreuz formuliert.

Das Volumenverhältnis der. Pasten kann hierbei im Bereich von 1 : 1 bis 20 1, vorzugsweise 4 : 1 bis 10 : 1 liegen. Die mengenmäßig größere Komponente wird im folgenden Basispaste genannt, die kleinere Katalysatorpaste. Die Komponenten können aber auch über drei oder mehr Pasten verteilt vorliegen. Bei geeigneter Wahl des Katalysatorsystems sind auch einkomponentige Formulierungen möglich.

Die Basispaste kann die gesamten Mengen der Komponenten (b) und (f), weiterhin gegebenenfalls Teile der Mengen der Komponenten (c) und (d) enthalten.

Die Katalysatorpaste enthält gegebenenfalls die restlichen oder alle Mengen und/oder Bestandteile der Komponenten (c) und (d).

Die Komponente (e) kann in der Basis- oder Katalysatorpaste enthalten sein oder auch über beide Pasten verteilt sein. Die Komponente (a) ist in der Basispaste enthalten oder über Basis- und Katalysatorpaste verteilt.

Die Vermischung kann durch statische oder dynamische Mischverfahren erfolgen. Auch Handanmischung der Komponenten ist möglich, wobei die Komponenten beispielsweise in Schraubtuben oder Tuben gelagert, manuell, beispielsweise Ober Stranglängenvergleich, abgemessen und vermischt werden.

Eine im Rahmen der Erfindung besonders bevorzugte Ausführungsform ist die Darreichung als Zweikomponentensystem mit statischen Mischaufsatz.

Eine weitere bevorzugte Ausführungsform der Erfindung betrifft ein Kit zur Herstellung eines Dentalmaterials, enthaltend entweder kein oder mindestens ein Applikationsgerät zur Ausbringung von Dentalmaterialien, kein oder mindestens einen statischen Mischaufsatz, mindestens eine Kartusche mit mindestens zwei Kammern, die mit einer erfindungsgemäßen Zusammensetzung gefüllt sind.

Hierbei sind die Komponenten beispielsweise wie folgt verteilt:
- Basispaste:
   Komponente Teile von (a), (b), Teile von (c), Teile von (e), (f)
- Katalysatorpaste:
   Komponente Teile von (a), Teile von (c), (d), Teile von (e).

Erfindungsgemäß hergestellte Formkörper können beispielsweise eine Biegefestigkeit, gemessen nach dem 4-Punkt-Biegeversuch, von über 40 MPa, bevorzugt von 70 bis 150 MPa aufweisen.

Die Bruchanfälligkeit der erfindungsgemäßen Formkörper liegt beispielsweise bei einem Dauerlasttest von 100.000 Belastungen mit 100 N bei maximal 40 %.

Im folgenden wird die Erfindung anhand von Beispielen näher beschrieben, wobei die Erfindung in keiner Weise durch die Beispiele limitiert werden soll.

### Herstellungsbeispiele

Aus den im folgenden aufgeführten Bestandteilen der Herstellungsbeispiele 1 bis 5 werden jeweils 100 g Basis- und 10 g Katalysatorpaste geknetet. Ein Teil hiervon wird in 10 : 1 -Kartuschen der Firma Mixpack, Rotkreuz gefüllt. Zur Anwendung werden sie mittels eines Dispensers durch eine statische Mischvorrichtung gedrückt und dabei vermischt. Die Aushärtung findet innerhalb von einigen Minuten statt.

**Herstellungsbeispiel 1:**

| | **Basis 1** | **Menge** | | | **Katalysator 1** | **Menge** | |
|---|---|---|---|---|---|---|---|
| Komponente | | g | Gew.-% | Komponente | | g | Gew.-% |
| a) | Dentalglaspulver (0̸<12µm) silanisiert mit Methacryloxypropyltrimethox ysilan | 25 | 25 | a) | Dentalglaspulver (0̸<12µm) | 3,4 | 34 |
| a) | mikrofeine Kieselsäure silanisiert (HDKH 2000, Wacker, Burghausen). | 5 | 5 | a) | mikrofeine Kieselsäure silanisiert (HDKH 2000, Wacker, Burghausen) | 0,7 | 7 |
| c) | Bis-(1-phenylpentan-1,3-dionato)-kupfer(II) | 0,00775 | 0,00775 | c) | 1-Benzyl-5-phenylbarbitursäure | 0,1 | 1 |
| c) | (β-Phenylethyl)-dibutyl-ammonium-chlorid | 0,352 | 0,352 | c) | 3,5,5-Trimethylhexansäure-tertiärbutylester | 0,06 | 0,6 |
| b) | 2,2-Bis-4(acryloxy-pentaethylenglycol)-phenylpropan | 13,77 | 13,77 | d) | 2,2-bis-4-(2-hydroxyethoxyphenyl)-propan-bis-acetat | 5,74 | 57,4 |
| f) | Genomer 4215 (Rahn AG, Zürich) | 6,89 | 6,89 | | | | |
| f) | Genomer 4205 (Rahn AG, Zürich) | 48,98025 | 48,98025 | | | | |

**Herstellungsbeispiel 2:**

| | **Basis 2** | **Menge** | | | **Katalysator 1** | **Menge** | |
|---|---|---|---|---|---|---|---|
| Komponente | | g | Gew.-% | Komponente | | g | Gew.-% |
| a) | Dentalglaspulver (0̸<12µm) silanisiert mit Methacryl-oxypropyltrimethoxysilan | 25 | 25 | a) | Dentalglaspulver (0̸<12µm) | 3,4 | 34 |
| a) | mikrofeine Kieselsäure silanisiert (HDKH 2000, Wacker, Burghausen) | 5 | 5 | a) | mikrofeine Kieselsäure silanisiert (HDKH 2000, Wacker, Burghausen) | 0,7 | 7 |
| c) | Bis-(1-phenylpentan-1,3-dionato)-kupfer(II) | 0,00775 | 0,00775 | c) | 1-Benzyl-5-phenylbarbitursäure | 0,1 | 1 |
| c) | (β-Phenylethyl)-dibutyl-ammonium-chlorid | 0,352 | 0,352 | c) | 3,5,5-Trimethylhexansäure-tertiärbutylester | 0.06 | 0,6 |
| b) | TEGDMA | 13,77 | 13,77 | d) | 2,2-Bis-4-(2-hydroxyethoxyphenyl)-propan-bis-acetat | 5,74 | 57.4 |
| f) | U 98-446 (Rahn AG, Zürich) | 13,77 | 13,77 | | | | |
| f) | Genomer 4205 (Rahn AG, Zürich) | 42,10025 | 42,10025 | | | | |

**Herstellungsbeispiel 3:**

| | **Basis 3** | **Menge** | | | **Katalysator 1** | **Menge** | |
|---|---|---|---|---|---|---|---|
| Komponente | | g | Gew.-% | Komponente | | g | Gew.-% |
| a) | Dentalglaspulver (0̸<12µm) silanisiert mit Methacryl-oxypropyltrimethoxysilan | 25 | 25 | a) | Dentalglaspulver (0̸<12µm) | 3,4 | 34 |
| a) | mikrofeine Kieselsäure silanisiert (HDKH 2000, Wacker, Burghausen) | 5 | 5 | a) | mikrofeine Kieselsäure silanisiert (HDKH 2000, Wacker, Burghausen) | 0,7 | 7 |
| c) | Bis-(1 -phenylpentan-1,3-dionato)-kupfer(II) | 0,00775 | 0,00775 | c) | 1-Benzyl-5-phenylbarbitursäure | 0,1 | 1 |
| c) | (β-Phenylethyl)-dibutyl-ammonium-chlorid | 0,352 | 0.352 | c) | 3,5,5-Trimethylhexansäure-tertiärbutylester | 0,06 | 0,6 |
| b) | TEGDMA | 10,33 | 10,33 | d) | 2,2-Bis-4-(2-hydroxyethoxyphenyl)-propan-bis-acetat | 5,74 | 57.4 |
| f) | Ebecryl 230 (UCB, Kerpen) | 10,33 | 10.33 | | | | |
| f) | 7,7,9-Trimethyl-4,13-dioxo-3,14,-dioxa-5,12-diazahexadecan-1,16-dioxy-dimethacrylat | 48,98025 | 48,98025 | | | | |

**Herstellungsbeispiel 4:**

| | **Basis 4** | **Menge** | | | **Katalysator 1** | **Menge** | |
|---|---|---|---|---|---|---|---|
| Komponente | | g | Gew.-% | Komponente | | g | Gew.-% |
| a) | Dentalglaspulver (0̸<3µm) silanisiert mit Methacryl-oxypropyltrimethoxysilan | 25 | 25 | a) | Dentalglaspulver (0̸<12µm) | 3,4 | 34 |
| a) | mikrofeine Kieselsäure silanisiert (HDKH 2000, Wacker, Burghausen) | 5 | 5 | a) | mikrofeine Kieselsäure silanisiert (HDKH 2000, Wacker, Burghausen) | 0,7 | 7 |
| c) | Bis-(1-phenylpentan-1,3-dionato)-kupfer(II) | 0,00775 | 0,00775 | c) | 1-Benzyl-5-phenylbarbitursäure | 0,1 | 1 |
| c) | (β-Phenylethyl)-dibutyl-ammonium-chlorid | 0,352 | 0.352 | c) | 3,5,5-Trimethylhexansäure-tertiärbutylester | 0,06 | 0,6 |
| b) | 2,2-Bis-4(acryloxy-pentaethylenglycol)-phenylpropan | 13,77 | 13,77 | d) | 2,2-Bis-4-(2-hydroxyethoxyphenyl)-propan-bis-acetat | 5,74 | 57,4 |
| f) | U 98-446 (Rahn AG, Zürich) | 6,89 | 6,89 | | | | |
| | 7,7,9-Trimethyl-4,13-dioxo-3,14,-dioxa-5,12-diazahexadecan-1,16-dioxy-dimethacrylat | 48,98025 | 48,98025 | | | | |

**Herstellungsbeispiel 5:**

| | **Basis 5** | **Menge** | | | **Katalysator 1** | **Menge** | |
|---|---|---|---|---|---|---|---|
| Komponente | | g | Gew.-% | Komponente | | g | Gew.-% |
| a) | Dentalglaspulver (0̸<1µm) silanisiert mit Methacryl-oxypropyltrimethoxysilan | 25 | 25 | a) | Dentalglaspulver (0̸<1µm) | 3.4 | 34 |
| a) | mikrofeine Kieselsäure silanisiert (HDKH 2000, Wacker, Burghausen) | 5 | 5 | a) | mikrofeine Kieselsäure silanisiert (HDKH 2000, Wacker, Burghausen) | 0,7- | 7 |
| c) | Bis-(1-phenylpentan-1,3-dionato)-kupfer(II) | 0,00775 | 0.00775 | c) | 1-benzoyl-5-phenylbarbitursäure | 0,1 | 1 |
| c) | (β-Phenylethyl)-dibutyl-ammonium-chlorid | 0,352 | 0,352 | c) | 3,5,5-Trimethylhexansäure-tertiärbutylester | 0,06 | 0.6 |
| b) | 2,2-Bis-4(acryloxy-pentaethylenglycol)-phenylpropan | 13,77 | 13,77 | d) | 2,2-Bis-4-(2-hydroxyethoxyphenyl)-propan-bis-acetat | 5,74 | 57,4 |
| f) | Ebecryl 270(UCB, Kerpen) | 10,33 | 10.33 | | | | |
| f) | 7,7,9-Trimethyl-4,13-dioxo-3,14,-dioxa-5,12-diazahexadecan-1,16-dioxy-dimethacrylat | 45,54025 | 45,54025 | | | | |

| | | | | | | | |
|---|---|---|---|---|---|---|---|
| 0̸: Durchmesser | | | | | | | |

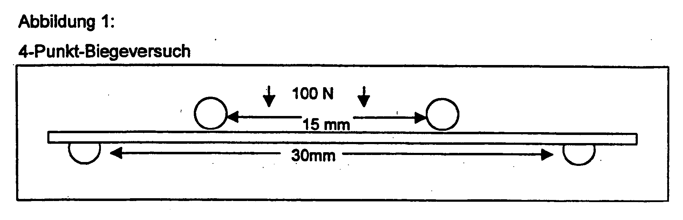

Es wurden Prüfkörper mit den Abmessungen 4 mm * 4 mm * 35 mm hergestellt und in einem Dauerlasttest gemäß Abbildung 1 im 4-Punkt-Biegeversuch bei 36°C in Wasser mit 100 N 100.000-mal belastet (eine Belastung pro Sekunde, Auflage im Abstand von 30 mm, Belastung im Abstand von 15 mm, maximal mögliche Durchbiegung der Probekörper von 1,4 mm durch die Apparatur vorgegeben).

Die erhaltenen Ergebnisse sind in nachfolgender Tabelle zusammengefasst:

| **Dentalmaterial** | **Biegefestigkeit [MPa] 4-Punkt-Biegeversuch** | **Dauerlasttest 100.000 Belastungen mit 100 N** |
|---|---|---|
| Vergleichsbeispiel 1: TRIM (Fa. Bosworth, Illinois) | 40 | 100% der Prüfkörper nach weniger als 10.000 Belastungen dauerhaft verformt |
| Vergleichsbeispiel 2: Flexspan (Fa. Jeneric/Pentron, Wallingford) | 70 | 100% der Prüfkörper gebrochen |
| Vergleichsbeispiel 3: Luxatemp Automix (Fa. DMG, Hamburg) | 85 | 75% der Prüfkörper gebrochen |
| Herstellungsbeispiel 1 (erfindungsgemäß) | 60 | 25% der Prüfkörper gebrochen |
| Herstellungsbeispiel 2 (erfindungsgemäß) | 50 | 35% der Prüfkörper gebrochen |
| Herstellungsbeispiel 3 (erfindungsgemäß) | 50 | 40% der Prüfkörper gebrochen |
| Herstellungsbeispiel 4 (erfindungsgemäß) | 70 | 25% der Prüfkörper gebrochen |
| Herstellungsbeispiel 5 (erfindungsgemäß) | 60 | 30% der Prüfkörper gebrochen |

Die erfindungsgemäßen Dentalmassen gemäß den Herstellungsbeispielen 1 bis 5 zeigen eine deutlich verringerte Bruchanfälligkeit bzw. verbesserte Formstabilität gegenüber herkömmlichen Materialien. Die erfindungsgemäßen Dentalmassen weisen neben der niedrigen Bruchanfälligkeit eine für provisorische K+B-Materialien genügende Biegefestigkeit auf.

## Patentansprüche

1. Zusammensetzung, enthaltend:
(a) 14 bis 60 Gew.-% Füllstoffe,
(b) 3 bis 35 Gew.-% mindestens einer einfach- oder mehrfach-ethylenisch ungesättigten Verbindung, die kein Urethan(meth)acrylat und ein Molekulargewicht zwischen 300 und 5000 g/mol aufweist,
(c) 0,1 bis 5 Gew.-% mindestens eines Initiatorsystems, das befähigt ist, eine radikalische Reaktion zu starten,
(d) 1 bis 40 Gew.-% Weichmacher mit einer Viskosität kleiner als 10 Pa·s bei 23°C (Kegel-Platte-Viskosimeter),
(e) 0 bis 10 Gew.-% Hilfsstoffe, wie Farbstoffe, Pigmente, Stabilisatoren, Lösungsmittel, rheologische Additive wie Fließverbesserer und/oder Verzögerer, und
(f) 30 bis 81,9 Gew.-% mindestens zwei im Molekulargewicht unterschiedliche Urethan(meth)acrylate, wobei diese in einem Molekulargewichtsverhältnis von 1,5 : 1 bis 50 : 1 vorliegen und das höhermolekulare Urethan(meth)acrylat ein Molekulargewicht zwischen 1.000 und 20.000 g/mol und das niedrigermolekulare Urethan(meth)acrylat ein Molekulargewicht zwischen 300 und 1.000 g/mol aufweisen.

2. Zusammensetzung gemäß Anspruch 1, wobei die Urethan(meth)acrylate der Komponente (f) mindestens 50 Gew.-% der gesamten ethylenisch ungesättigten Verbindungen [(b) + (f)] ausmachen.

3. Zusammensetzung nach einem der Ansprüche 1 oder 2, wobei die höhermolekularen Urethan(meth)acrylate ausgewählt sind aus der Gruppe: Polyether-, Polyester- und/oder Polycarbonat-Urethanoligomer(meth)acrylate.

4. Zusammensetzung nach einem der Ansprüche 1 bis 3, wobei es sich bei der Komponente (b) um Acrylsäure- und/oder Methacrylsäureester einer ethoxylierten und/oder propoxylierten Verbindung, eines Polyethers oder eines Alkylpolyols handelt.

5. Zusammensetzung nach einem der Ansprüche 1 bis 4, wobei die Komponente (d) ein Molekulargewicht zwischen 150 g/mol und 5.000g/mol aufweist.

6. Zusammensetzung nach einem der Ansprüche 1 bis 5, wobei diese als mindestens zweikomponentiges Paste-Paste-System in üblichen dentalen Applikationssystemen angemischt werden kann.

7. Zusammensetzung nach einem der Ansprüche 1 bis 5, wobei diese als einkomponentiges System formuliert wird.

8. Zusammensetzung nach einem der vorangehenden Ansprüche zur Anwendung im zahntechnischen oder zahnmedizinischen Bereich, insbesondere zur Herstellung von provisorischen Kronen, Brücken, Füllungen, Inlays und Onlays.

9. Kit zur Herstellung eines Dentalmaterials, enthaltend entweder kein oder mindestens ein Applikationsgerät zur Ausbringung von Dentalmaterialien, kein oder mindestens einen statischen Mischaufsatz, mindestens eine Kartusche mit mindestens zwei Kammern, die mit einer Zusammensetzung nach einem der Ansprüche 1 bis 7 gefüllt sind.

10. Verwendung von Zusammensetzungen, enthaltend:
(a) 14 bis 60 Gew.-% Füllstoffe,
(b) 3 bis 35 Gew.-% mindestens einer einfach- oder mehrfachethylenisch ungesättigten Verbindung, die kein Urethan(meth)acrylat ist und ein Molekulargewicht zwischen 300 und 5000 g/mol aufweist,
(c) 0,1 bis 5 Gew.-% mindestens eines Initiatorsystems, das befähigt ist, eine radikalische Reaktion zu starten,
(d) 1 bis 40 Gew.-% Weichmacher mit einer Viskosität kleiner als 10 Pa·s bei 23°C (Kegel-Platte-Viskosimeter)_{.}
(e) 0 bis 10 Gew.-% Hilfsstoffe, wie Farbstoffe, Pigmente, Stabilisatoren, Lösungsmittel, rheologische Additive wie Fließverbesserer und/oder Verzögerer, und
(f) 30 bis 81,9 Gew.-% mindestens zwei im Molekulargewicht unterschiedliche Urethan(meth)acrylate, wobei diese in einem Molekulargewichtsverhältnis von 1,5 : 1 bis 50 : 1 vorliegen und das höhermolekulare Urethan(meth)acrylat ein Molekulargewicht zwischen 1.000 und 20.000 g/mol und das niedrigermolekulare Urethan(meth)acrylat ein Molekulargewicht zwischen 300 und 1.000 g/mol aufweisen,
zur Herstellung von Dentalmaterialien mit einer Bruchanfälligkeit bei einem Dauerlasttest von 100.000 Belastungen mit 100 N von maximal 40 %.

11. Verwendung von Zusammensetzungen enthaltend:
(a) 14 bis 60 Gew.-% Füllstoffe,
(b) 3 bis 35 Gew.-% mindestens einer einfach- oder mehrfachethylenisch ungesättigten Verbindung, die kein Urethan(meth)acrylat ist und ein Molekulargewicht zwischen 300 und 5000 g/mol aufweist,
(c) 0,1 bis 5 Gew.-% mindestens eines Initiatorsystems, das befähigt ist, eine radikalische Reaktion auszulösen,
(d) 1 bis 40 Gew.-% Weichmacher mit einer Viskosität kleiner als 10 Pa·s bei 23°C (Kegel-Platte-Viskosimeter)_{.}
(e) 0 bis 10 Gew.-% Hilfsstoffe, wie Farbstoffe, Pigmente, Stabilisatoren, Lösungsmittel, rheologische Additive wie Ffießverbesserer und/oder Verzögerer, und
(f) 30 bis 81,9 Gew.-% mindestens zwei im Molekulargewicht unterschiedliche Urethan(meth)acrylate, wobei diese in einem Molekulargewichtsverhältnis von 1,5 : 1 bis 50 : 1 vorliegen und das höhermolekulare Urethan(meth)acrylat ein Molekulargewicht zwischen 1.000 und 20.000 g/mol und das niedrigermolekulare Urethan(meth)acrylat ein Molekulargewicht zwischen 300 und 1.000 g/mol aufweisen,
zur Herstellung von Dentalmaterialien mit einer Biegefestigkeit, gemessen nach dem 4-Punkt-Biegeversuch, von über 40 MPa.

## Claims

1. Composition containing:
(a) 14 to 60 wt.-% fillers,
(b) 3 to 35 wt.-% of at least one mono- or polyethylenically unsaturated compound which is not a urethane (meth)acrylate and has a molecular weight between 300 and 5000 g/mol,
(c) 0.1 to 5 wt.-% of at least one initiator system which is capable of starting a free-radical reaction,
(d) 1 to 40 wt.-% plasticizers having a viscosity of less than 10 Pa·s at 23°C (cone-plate viscometer),
(e) 0 to 10 wt.-% auxiliaries, such as dyes, pigments, stabilizers, solvents, rheological additives such as flow improvers and/or retarders, and
(f) 30 to 81.9 wt.-% of at least two urethane (meth)acrylates with different molecular weights, these being present in a molecular weight ratio of 1.5:1 to 50:1 and the higher molecular urethane (meth)acrylate having a molecular weight between 1000 and 20 000 g/mol and the lower molecular urethane (meth)acrylate having a molecular weight between 300 and 1000 g/mol.

2. Composition according to Claim 1, wherein the urethane (meth)acrylates of component (f) constitute at least 50 wt.-% of the total ethylenically unsaturated compounds [(b)+(f)].

3. Composition according to either of Claims 1 and 2, wherein the higher molecular urethane (meth)acrylates are selected from the group consisting of polyether, polyester and/or polycarbonate urethane oligomer (meth)acrylates.

4. Composition according to any one of Claims 1 to 3, wherein component (b) comprises acrylic and/or methacrylic esters of an ethoxylated and/or propoxylated compound, of a polyether or of an alkyl polyol.

5. Composition according to any one of Claims 1 to 4, wherein component (d) has a molecular weight between 150 g/mol and 5000 g/mol.

6. Composition according to any one of Claims 1 to 5, which is mixable as an at least two-component paste-paste system in customary dental application systems.

7. Composition according to any one of Claims 1 to 5, which is formulated as a one-component system.

8. Composition according to any one of the preceding claims for use in the field of dental engineering or dentistry, in particular for the preparation of provisional crowns, bridges, fillings, inlays and onlays.

9. Kit for the preparation of a dental material, containing either no or at least one application apparatus for the application of dental materials, no or at least one static mixing element, at least one cartridge with at least two chambers which are filled with a composition according to any one of Claims 1 to 7.

10. Use of compositions containing:
(a) 14 to 60 wt.-% fillers,
(b) 3 to 35 wt.-% of at least one mono- or polyethylenically unsaturated compound which is not a urethane (meth)acrylate and has a molecular weight between 300 and 5000 g/mol,
(c) 0.1 to 5 wt.-% of at least one initiator system which is capable of starting a free-radical reaction,
(d) 1 to 40 wt.-% plasticizers having a viscosity of less than 10 Pa·s at 23°C (cone-plate viscometer),
(e) 0 to 10 wt.-% auxiliaries, such as dyes, pigments, stabilizers, solvents, rheological additives such as flow improvers and/or retarders, and
(f) 30 to 81.9 wt.-% of at least two urethane (meth)acrylates with different molecular weights, these being present in a molecular weight ratio of 1.5:1 to 50:1 and the higher molecular urethane (meth)acrylate having a molecular weight between 1000 and 20 000 g/mol and the lower molecular urethane (meth)acrylate having a molecular weight between 300 and 1000 g/mol,
for the preparation of dental materials with a fracture susceptibility of not more than 40% in an endurance test of 100 000 loads of 100 N.

11. Use of compositions containing:
(a) 14 to 60 wt.-% fillers,
(b) 3 to 35 wt.-% of at least one mono- or polyethylenically unsaturated compound which is not a urethane (meth)acrylate and has a molecular weight between 300 and 5000 g/mol,
(c) 0.1 to 5 wt.-% of at least one initiator system which is capable of starting a free-radical reaction,
(d) 1 to 40 wt.-% plasticizers having a viscosity of less than 10 Pa·s at 23°C (cone-plate viscometer),
(e) 0 to 10 wt.-% auxiliaries, such as dyes, pigments, stabilizers, solvents, rheological additives such as flow improvers and/or retarders, and
(f) 30 to 81.9 wt.-% of at least two urethane (meth)acrylates with different molecular weights, these being present in a molecular weight ratio of 1.5:1 to 50:1 and the higher molecular urethane (meth)acrylate having a molecular weight between 1000 and 20 000 g/mol and the lower molecular urethane (meth)acrylate having a molecular weight between 300 and 1000 g/mol,
for the preparation of dental materials with a bending strength, measured according to the 4-point bending test, of over 40 MPa.

## Revendications

1. Composition contenant :
(a) 14 à 60 % en poids de charges,
(b) 3 à 35 % en poids d'au moins un composé à une ou plusieurs insaturations éthyléniques, qui n'est pas un uréthanne(méth)acrylate et présente une masse moléculaire comprise entre 300 et 5 000 g/mole,
(c) 0,1 à 5 % en poids d'au moins un système amorceur qui est apte à déclencher une réaction radicalaire,
(d) 1 à 40 % en poids de plastifiant ayant une viscosité inférieure à 10 Pa.s à 23°C (viscosimètre à bille-plateau),
(e) 0 à 10 % en poids d'adjuvants, tels que des colorants, des pigments, des stabilisants, des solvants, des additifs rhéologiques tels que des agents améliorant l'écoulement et/ou des ralentisseurs, et
(f) 30 à 81,9 % en poids d'au moins deux uréthanne(méth)acrylates qui diffèrent quant à la masse moléculaire, ces derniers se trouvant en un rapport de masses moléculaires allant de 1,5:1 à 50:1 et l'uréthanne(méth)acrylate de masse moléculaire plus élevée ayant une masse moléculaire comprise entre 1 000 et 20 000 g/mole et l'uréthanne(méth)acrylate ayant la masse moléculaire plus faible ayant une masse moléculaire comprise entre 300 et 1 000 g/mole.

2. Composition selon la revendication 1, dans laquelle les uréthanne(méth)acrylates du composant (f) constituent au moins 50 % en poids de la totalité des composés [(b) + (f)] à insaturation éthylénique.

3. Composition selon la revendication 1 ou 2, dans laquelle les uréthanne(méth)acrylates de masse moléculaire plus élevée sont choisis dans le groupe constitué par des oligomères polyéther-, polyester- et/ou polycarbonate-uréthanne(méth)-acrylates.

4. Composition selon l'une quelconque des revendications 1 à 3, dans laquelle le composant (b) consiste en un ester d'acide acrylique et/ou d'acide méthacrylique d'un composé éthoxylé et/ou propoxylé, d'un polyéther ou d'un alkylpolyol.

5. Composition selon l'une quelconque des revendications 1 à 4, dans laquelle le composant (d) a une masse moléculaire comprise entre 150 g/mole et 5 000 g/mole.

6. Composition selon l'une quelconque des revendications 1 à 5, dans laquelle celle-ci peut être incorporée, sous forme de système pâte-pâte au moins bicomposant, dans des systèmes d'application dentaires usuels.

7. Composition selon l'une quelconque des revendications 1 à 5, dans laquelle celle-ci est formulée sous forme de système monocomposant.

8. Composition selon l'une quelconque des revendications précédentes, pour utilisation dans le domaine technique dentaire ou de la médecine dentaire, en particulier pour la fabrication de couronnes provisoires, de bridges, de plombages, d'inlays (incrustations) et d'onlays (incrustations de surface).

9. Nécessaire pour la fabrication d'un matériau dentaire, soit ne contenant pas d'applicateur, soit en contenant au moins un pour l'application de matériaux dentaires, ne contenant pas de dispositif de mélange statique ou en contenant au moins un, contenant au moins une cartouche comportant au moins deux chambres qui sont garnies d'une composition selon l'une quelconque des revendications 1 à 7.

10. Utilisation de compositions contenant :
(a) 14 à 60 % en poids de charges,
(b) 3 à 35 % en poids d'au moins un composé à une ou plusieurs insaturations éthyléniques, qui n'est pas un uréthanne(méth)acrylate et présente une masse moléculaire comprise entre 300 et 5 000 g/mole,
(c) 0,1 à 5 % en poids d'au moins un système amorceur qui est apte à déclencher une réaction radicalaire,
(d) 1 à 40 % en poids de plastifiant ayant une viscosité inférieure à 10 Pa.s à 23 °C (viscosimètre à bille-plateau),
(e) 0 à 10 % en poids d'adjuvants, tels que des colorants, des pigments, des stabilisants, des solvants, des additifs rhéologiques tels que des agents améliorant l'écoulement et/ou des ralentisseurs, et
(f) 30 à 81,9 % en poids d'au moins deux uréthanne(méth)acrylates qui diffèrent quant à la masse moléculaire, ces derniers se trouvant en un rapport de masses moléculaires allant de 1,5:1 à 50:1 et l'uréthanne(méth)acrylate de masse moléculaire plus élevée ayant une masse moléculaire comprise entre 1 000 et 20 000 g/mole et l'uréthanne(méth)acrylate ayant la masse moléculaire plus faible ayant une masse moléculaire comprise entre 300 et 1 000 g/mole.
pour la préparation de matériaux dentaires ayant une tendance à la rupture, dans un essai de sollicitation de longue durée, de 100 000 sollicitations à 100 N, d'au maximum 40 %.

11. Utilisation de compositions contenant :
(a) 14 à 60 % en poids de charges,
(b) 3 à 35 % en poids d'au moins un composé à une ou plusieurs insaturations éthyléniques, qui n'est pas un uréthanne(méth)acrylate et présente une masse moléculaire comprise entre 300 et 5 000 g/mole,
(c) 0,1 à 5 % en poids d'au moins un système amorceur qui est apte à déclencher une réaction radicalaire,
(d) 1 à 40 % en poids de plastifiant ayant une viscosité inférieure à 10 Pa.s à 23 °C (viscosimètre à bille-plateau),
(e) 0 à 10 % en poids d'adjuvants, tels que des colorants, des pigments, des stabilisants, des solvants, des additifs rhéologiques tels que des agents améliorant l'écoulement et/ou des ralentisseurs, et
(f) 30 à 81,9 % en poids d'au moins deux uréthanne(méth)acrylates qui diffèrent quant à la masse moléculaire, ces derniers se trouvant en un rapport de masses moléculaires allant de 1,5:1 à 50:1 et l'uréthanne(méth)acrylate de masse moléculaire plus élevée ayant une masse moléculaire comprise entre 1 000 et 20 000 g/mole et l'uréthanne(méth)acrylate ayant la masse moléculaire plus faible ayant une masse moléculaire comprise entre 300 et 1 000 g/mole.
pour la préparation de matériaux dentaires ayant une résistance à la flexion, mesurée selon l'essai de flexion en 4 points, de plus de 40 MPa.
